(19) **Europäisches Patentamt**
**European Patent Office**
**Office européen des brevets**

(11) Veröffentlichungsnummer: **0 340 491**
**A1**

(12) # EUROPÄISCHE PATENTANMELDUNG

(21) Anmeldenummer: 89106465.1

(22) Anmeldetag: 12.04.89

(51) Int. Cl.4: **A23L 1/236 , A23C 9/12 , C13F 3/00 , C13K 13/00**

(30) Priorität: 09.05.88 DE 3815794
06.05.88 DE 3815513

(43) Veröffentlichungstag der Anmeldung:
08.11.89 Patentblatt 89/45

(84) Benannte Vertragsstaaten:
AT BE CH DE ES FR GB GR IT LI LU NL SE

(71) Anmelder: **Biodyn AG**
**Industriestr. 31**
**CH-8305 Dietlikon(CH)**

(72) Erfinder: **Lembke, Andreas, Prof. Dr. Dr.**
**Eutiner Strasse 1**
**D-2420 Eutin-Sielbeck(DE)**
Erfinder: **Strobel, Hans Joachim, Dr.**
**Opfikoner Strasse 3**
**CH-8303 Bassersdorf(CH)**

(74) Vertreter: **Hach, Hans Karl, Dr.**
**Tarunstrasse 23**
**D-6950 Mosbach-Waldstadt(DE)**

(54) Süssungsmittel für Nahrungsmittel.

(57) Saccharose ist zum Zahnschutz gegen Karies mit Galactose versetzt.

EP 0 340 491 A1

## SÜSSUNGSMITTEL FÜR NAHRUNGSMITTEL

Die Erfindung betrifft ein Süßungsmittel für Nahrungsmittel, das im wesentlichen aus Saccharose besteht.

Das am weitesten verbreitete Süßungsmittel für Nahrungsmittel ist Saccharose. Es ist bekannt, daß durch Saccharose die Bildung von Karies begünstigt wird, weil die Saccharose ein hervorragendes Nahrungsmittel für die säurebildenden, Karies hervorrufenden Bakterien ist und zudem diesen Bakterien auch noch nach der Nahrungsaufnahme zur Verfügung steht, weil die Saccharose dazu neigt, an den Zähnen zu haften. Entsprechendes gilt, wenn auch mit etwas geringerer Wirkung, auch für Glucose und Fructose.

Dem wirkt, wie aus der EU 01844121/A2 bekannt, ein Zusatz von Galactose in zuckerhaltigen Nahrungsmitteln entgegen, weil die Galactose die Rezeptoren belegt, mit denen die Karies hervorrufenden Bakterien an der Zahnoberfläche haften.

Aufgabe der Erfindung ist es, ein Süßungsmittel der eingangs genannten Art so auszugestalten, daß die Karies fördernde Wirkung vermieden wird.

Die Erfindung ist dadurch gekennzeichnet, daß auf 100 Trockengewichtsteile (TGT) Saccharose 1,5 bis 20 TGT, vorzugsweise 3 bis 7 TGT, Galactose zugemischt sind.

Durch den Galactose-Zusatz ist sichergestellt, daß beim Einsatz des Süßungsmittels im Nahrungsmittel eine entsprechende Menge Galactose in das Nahrungsmittel gelangt und die angestrebte, zahnschützende Wirkung erzielt wird. Der Zusatz an Galactose ist dabei mengenmäßig so bemessen, daß er für die eingesetzte Saccharose die optimale zahnschützende Wirkung bei minimalen Zusatzmengen an Galactose bietet.

Die Galactose wird vorzugsweise in reiner Form zugesetzt. Sie kann aber auch wirtschaftlich vorteilhaft eingesetzt werden in Form eines Lactosehydrolysationsprodukts (LHP), das gewonnen ist, indem reine Lactose oder Molke mit Beta-Galactosidase - durch unmittelbaren Einsatz von Mikroorganismen, die diese Beta-Galactosidase herstellen oder durch Einsatz eines Beta-Galactosidase enthaltenden Enzympräparates - behandelt wird, so daß die Lactose wenigstens zu einem Teil, vorzugsweise zu 40 bis 85% zu Galactose und Glucose hydrolysiert wird.

Molkeeiweiß hat sich in dem LHP in vielen Fällen als nachteilig erwiesen, weil es zum Beispiel bei Getränken zu einer unerwünschten Trübung führt. Vor allem aber ist nachteilig, daß die angestrebte Schutzwirkung der Galactose durch Molkeeiweiß behindert werden kann. Dem trägt eine Weiterbildung der Erfindung Rechnung, nach der beim Einsatz von Molke als Ausgangsprodukt zur Gewinnung des zum Zahnschutz bestimmten LHP das in dem Ausgangsprodukt enthaltene Protein entfernt wird.

Das kann geschehen, indem man von vornherein von entsprechend proteinfreier Molke ausgeht oder in der Molke vor der Hydrolysation oder nach der Hydrolysation die Proteine entfernt, vorzugsweise durch Ausfällen und Abfiltern.

Absolute Proteinfreiheit ist in diesem Zusammenhang nicht erforderlich und auch nicht gemeint und mit tragbarem Aufwand auch nicht möglich. Gemeint ist relative Proteinfreiheit, so wie sie großtechnisch wirtschaftlich erzielbar ist mit einem Restproteingehalt nicht größer als 0,5%.

Die in dem LHP außer dem Protein eventuell vorhandenen Substanzen, nämlich die Salze aus der Molke beziehungsweise restliche Lactose und die bei der Hydrolysation neben der Galactose entstehende Glucose, sind in den hier in Frage stehenden Mengen als Zusatz zu Nahrungsmitteln unschädlich und auch in geschmacklicher und sonstiger Hinsicht nicht nachteilig; im Gegenteil sie enthalten als Nährstoffe nützliche Lactose und Glucose und, wenn von Molke ausgegangen wird, außerdem noch wünschenswerte Mineralsalze.

Die im LHP eventuell enthaltenen Rückstände von zur Hydrolysation eingesetzten Mikroorganismen werden vorzugsweise entfernt, zweckmäßig in wässriger Lösung des LHP.

Galactose beziehungsweise das LHP sind wasserlöslich. Deshalb kann das Süßungsmittel sehr einfach aus wässriger Lösung, zum Beispiel Sirup, bestehen, der Galactose beziehungsweise das LHP beigelöst ist.

Wenn das Süßungsmittel in trockener Form bereitgestellt werden soll, kann man die im Verhältnis zur Saccharose schwerere Löslichkeit der Bestandteile des LHP in Betracht ziehen. Diesem Umstand tragen Weiterbildungen der Erfindung Rechnung, indem die zugesetzte Galactose beziehungsweise das LHP in möglichst feiner molekularer Verteilung eingesetzt wird, durch die das In-Lösung-Gehen begünstigt wird.

Eine erste dementsprechende Weiterbildung ist dadurch gekennzeichnet, daß die Saccharose sowie Galactose beziehungsweise LHP enthaltende Lösung gebildet und dann eingetrocknet wird und daß das Trockenprodukt dann gemahlen wird. Die Galactose beziehungsweise das LHP durchsetzt dann im gemahlenen Trockenprodukt in feinster Verteilung die Saccharose und gerät auf diese Weise, wenn die Saccharose bei Gebrauch, also wenn sie einem Nahrungsmittel zugesetzt wird, in Lösung geht, in dieser feinsten Verteilung ebenfalls in das Lösungsmittel und damit auch schnell in Lösung.

Eine zweite Weiterbildung ist dadurch gekennzeichnet, daß die Galactose beziehungsweise das LHP den Körnern von feinkörniger kristalliner Saccharose in feinster Verteilung aufgeschichtet ist. Eine solche Aufschichtung kann man beispielsweise dadurch erzielen, daß man die Körnchen aus Saccharose im Wasserdampf oberflächlich anlöst und dann die Galactose aufsprüht unter gleichzeitigem Umrühren.

Eine dritte Weiterbildung ist dadurch gekennzeichnet, daß feinkörniger kristalliner Saccharose Galactose beziehungsweise LHP in einer Körnchengröße von 0,01 bis 0,1 mm untermischt ist. Durch die besonders feine Körnchengröße ist sicherge- stellt, daß die Galactose nur wenig verzögert gegenüber den Körnchen aus Saccharose in Lösung geht. feinvermahlener kristalliner Saccharose feinvermahlenes galactosehaltiges Pulver zugesetzt ist, daß dieses Galactose-Pulver gewonnen ist, indem auf 100 TGT Galactose oder LHP 150 bis 400 TGT wasserlöslicher Füllstoff, vorzugsweise Glycin, und 10bis 40 TGT Löslichkeitsbeschleuniger, vorzugsweise Natriumcarboxymethylcellulose in Wasser gut gemischt gelöst, eingetrocknet und dann gemahlen wird.

Der Füllstoff Glycin ist in dem Mahlgut feinkörnig und deshalb schnell löslich. Er ist mit der Galactose beziehungsweise dem LHP in feinster Form durchsetzt, so daß dieses mit dem Glycin beschleunigt in Lösung geht. Der Löslichkeitsbeschleuniger beschleunigt das Inlösunggehen des Glycins.

Die Erfindung wird nun anhand einiger Beispiele näher erläutert.

## BEISPIEL 1

Saccharosesirup mit einem Wassergehalt von 20 % und wässrige Galactoselösung werden vermischt. Der Einsatz erfolgt mit 1 kg (Kilogramm) Trockengewicht Saccharose und 50 g Trockengewicht Galactose. Die Mischung wird in einem Rotationsverdampfer eingeengt. Die nicht kristallisierte, noch leicht wasserhaltige Mischung wird durch Auftragen auf eine Kühlwalze zum Erstarren gebracht. Die erstarrte Schmelze wird in einem Brecher zerkleinert und anschließend in Partikel mit einem Durchmesser von 1 bis 2 mm (Millimeter) gemahlen. Das entstehende Pulver wird durch Siebfraktionierung in spezielle Fraktionen aufgeteilt.

## BEISPIEL 2

1 kg (Kilogramm) Saccharosesirup mit einem Wassergehalt von 20% (Prozent) werden 40 g (Gramm) reine Galactose, die in Wasser bis zur Sättigung gelöst ist, untermischt. Dann wird der Sirup wieder auf die ursprüngliche Konzentration eingeengt.

## BEISPIEL 3

Saccharosekristalle werden durch Sieben nach Kristallgrößenklassen getrennt. Von der Fraktion mit Korngröße kleiner als 1 mm wird 1 kg in einem Dragierkessel vorgelegt und unter ständigem Rotieren wird intermittierend eine wässrige Galactoselösung mit insgesamt 55 g Trockengewicht Galactose aufgesprüht.

## BEISPIEL 4

1 kg körnige, kristalline Saccharose mit einer Korngröße von 0,2 bis 1,8 mm wird unter ständigem, intensivem Rühren von gesättigtem Wasserdampf durchblasen, bis die Saccharosekörnchen oberflächlich angelöst sind. Gleichzeitig mit dem Wasserdampf werden 55 g staubförmig feingemahlenes Galactosepulver in feinster Verteilung in die Masse eingeblasen. Sobald die Galactose sich in den weichgewordenen Oberflächenschichten der Saccharosekörnchen gelöst hat, wird die Dampfbeschickung eingestellt und stattdessen die Masse mit Heißluft durchblasen und es wird weitergerührt, bis die Körnchen getrocknet sind.

## BEISPIEL 5

1 kg feinkörniger, kristalliner Saccharose werden 7 g auf ein Korngröße von 0,01 bis 0,1 mm gemahlene Galactose untermischt.

BEISPIEL 6

Die Mischung nach BEISPIEL 5 wird unter ständigem Rühren mit gesättigtem Wasserdampf durchblasen, bis die Saccharosekörnchen oberflächlich angelöst sind und die Galactoseteilchen aufgenommen haben. Im Anschluß daran wird mit Heißluft durchblasen und weitergerührt, bis die Saccharosekörnchen auch oberflächlich wieder ausgehärtet sind.

BEISPIEL 7

Es wird ein Galactose-Pulver gewonnen, indem 1 kg Galactose, 1,5 kg Glycin und 10 g Natriumcarboxymethylcellulose in 4 l (Liter) Wasser gelöst und gut gemischt werden. Die Lösung wird im Wasserbad eingetrocknet und der Trockenrückstand dann gemahlen bis zu einer Körnchengröße von 0,05 bis 0,5 mm. 1 kg körnige kristalline Saccharose werden 80 g dieses Galactose-Pulvers zugesetzt.

BEISPIEL 8

Herstellung von LHP aus Lactose

Eingesetzte Lactose

10 kg (Kilogramm) Lactosepulver wird in 30 l (Liter) 18°C warmem Wasser gelöst. Der gewonnenen Lösung werden 70 l 70°C warmes Wasser zugesetzt sowie 100 g (Gramm) Ascorbinsäure.

pH-Wert-Einstellen

Die Lösung wird auf 20°C abgekühlt und mit Milchsäure auf pH 4,5 eingestellt.

Hydrolysieren

Die Lösung wird bei einer Temperatur von 32°C mit 3 g des Enzyms EC 3.2.1.23 versetzt und 40 Stunden unter Rühren auf 32°C gehalten. Dann sind ca. 75 % TM der Lactose hydrolysiert.

LHP Einsatzfertig

Die hydrolysierte Lösung wird durch Getriertrocknen auf 99 % TM eingetrocknet. Das so gewonnene LHP-Pulver enthält an Trockensubstanz:

| Lactose | 25 % |
|---|---|
| Galactose | 39 % |
| Glucose | 36 %. |

BEISPIEL 9

4

Herstellung von LHP aus Molkepulver

Eingesetzte Molke

Molkepulver wird gewonnen durch Trocknen von bei der Käseherstellung gewonnener Molke. Das Molkepulver hat folgende Trockenbestandteile: Protein 3,3 %; Fett 1,4 %; Lactose 84,3 %; Mineralstoffe 8,2 %. 10 kg Molkepulver wird in 30 l 18°C warmem Wasser gelöst. Der gewonnenen Lösung werden 70 l 70°C warmes Wasser zugesetzt, sowie 100 g Ascorbinsäure.

Proteinabscheiden

Die Molke wird 30 Minuten bei 70°C gerührt, das dabei aus fallende Protein wird abfiltriert. Das Filtrat wird dann nochmals 30 Minuten bei 70°C gerührt und dann auf 20°C abgekühlt.

pH Wert Einstellen

Das abgekühlte Filtrat wird mit Milchsäure auf pH 4,5 eingestellt.

Hydrolysieren

Das Filtrat wird bei einer Temperatur von 32°C mit 3 g des Enzyms EC 3.2.1.23 versetzt. Die Lösung wird 40 Stunden unter Rühren auf 32°C gehalten. Dann sind ca. 80 % TM der Lactose hydrolysiert.

LHP Einsatzfertig

Die hydrolysierte Lösung wird durch Gefriertrocknen auf 99 % TM eingetrocknet. Das so gewonnene LHP Pulver enthält an Trockensubstanz:

| | |
|---|---|
| Protein | 0,5 % |
| Fett | 1,5 % |
| Lactose | 21,8 % |
| Galactose | 35,0 % |
| Glucose | 33,0 % |
| Mineralstoffe | 8,2 %. |

Beispiel 10

Herstellung von LHP aus Molkepulver

Eingesetzte Molke

wie Beispiel 9

Proteinabscheiden

wie Beispiel 9

5

pH Wert Einstellen

wie Beispiel 9

## Hydrolysieren

Das Filtrat wird pasteurisiert und dann auf eine Temperatur von 32 °C gebracht und die Lösung wird dann mit Lactobacillus Leichmannii geimpft. Die Lösung wird 40 Stunden auf 32 °C gehalten. Dann sind ca. 75 % TM der Lactose hydrolysiert. Der Lactobacillus Leichmannii wird vorzugsweise vor dem Impfen auf die Lactosehydrolysation konditioniert. Eingesetzt wird vorzugsweise der unter DSM 20076 oder DSM 20355 bekannte Stamm.

## LHP Einsatzfertig

Aus der hydrolysierten Lösung werden die Mikroorganismen abfiltriert und das Filtrat wird durch Gefriertrocknen auf 99 % TM eingetrocknet. Das so gewonnene LHP Pulver enthält an Trockensubstanz:

| Protein | 0,5 % |
|---|---|
| Fett | 1,5 % |
| Lactose | 25,0 % |
| Galactose | 39,0 % |
| Glucose | 36,0 % |
| Mineralstoffe | 8,2 % |

Die Beispiele 1 bis 7 sind abänderbar, indem statt der eingesetzten Saccharose die gleiche Menge an Fructose oder Glucose oder Mischungen aus Saccharose, Fructose und/oder Glucose eingesetzt werden.

Die Beispiele 1 bis 7 sind abänderbar, indem zusätzlich zur Galactose künstlicher Süßstoff, wie Cyclamat, Saccharinat und/oder Aspartame, zugesetzt wird, und zwar vorzugsweise mit der gleichen Technik wie die Galactose zugesetzt wird. Die Menge an zugesetztem künstlichen Süßstoff wird dabei vorzugsweise so gewählt, daß sich für das fertige Produkt die Süßkraft von kristalliner Saccharose ergibt.

Die vorgenannten Beispiele sind weiter abänderbar, indem die eingesetzte Menge an Galactose geändert wird. Nach diesen Abänderungen kommen beispielsweise vorteilhaft in Betracht: bei den Beispielen 1, 2, 3, 4 und 5: 15 g, 30 g, 70 g oder 200 g Trockengewicht Galactose anstelle der in den betreffenden Beispielen angegebenen Galactose-Mengen.

Die Beispiele 1 bis 7 und die vorgenannten Abänderungen dieser Beispiele sind weiter abänderbar, indem die dort angegebenen Galactosemengen, statt in Form reiner Galactose, in Form eines LHP, das nach Beispiel 8 oder 9 gewonnen ist, zugesetzt werden.

## Ansprüche

1. Süßungsmittel für Nahrungsmittel, das im wesentlichen aus Saccharose besteht, dadurch gekennzeichnet,
daß auf 100 Trockengewichtsteile (TGT) Saccharose 1,5 bis 20 TGT, vorzugsweise 3 bis 7 TGT, Galactose zugemischt sind.

2. Süßungsmittel nach Anspruch 1, dadurch gekennzeichnet,
daß die Galactose in reiner Form eingesetzt ist.

3. Süßungsmittel nach Anspruch 1, dadurch gekennzeichnet,
daß die Galactose in Form eines Lactosehydrolysationsprodukts (LHP) zugesetzt ist und
daß das LHP gewonnen ist, indem Lactose mit Beta-Galactosidase hydrolysiert ist und das sich so ergebende Produkt mit seinem gesamten Zuckergehalt als LHP eingesetzt ist.

4. Süßungsmittel nach Anspruch 1, dadurch gekennzeichnet,
daß Molke mit Beta-Galactosidase - durch unmittelbaren Einsatz von Mikroorganismen, die diese Beta-Galactosidase herstellen oder durch Einsatz eines Beta-Galactosidase enthaltenden Enzympräparates - behandelt und dadurch in der Molke enthaltene Lactose hydrolysiert wird,

6

daß - um ein proteinfreis LHP zu gewinnen - von proteinfreier Molke ausgegangen wird oder aus der Molke vor der Hydrolysation und/oder nach der Hydrolysation die Proteine entfernt werden, vorzugsweise durch Ausfällen und Abfiltrieren, und

daß das sich so ergebende Produkt mit seinem gesamten Zuckergehalt als LHP eingesetzt ist.

5. Süßungsmittel nach Anspruch 4, dadurch gekennzeichnet,

daß eine die Saccharose sowie Galactose beziehungsweise LHP enthaltende Lösung gebildet und dann eingetrocknet wird und

daß das Trockenprodukt dann gemahlen wird.

6. Süßungsmittel nach einem der Ansprüche 1 bis 4, dadurch gekennzeichnet,

daß die Galactose beziehungsweise das LHP den Körnern von feinkörniger kristalliner Saccharose in feinster Verteilung aufgeschichtet ist.

7. Süßungsmittel nach einem der Ansprüche 1 bis 4, dadurch gekennzeichnet,

daß feinkörniger kristalliner Saccharose Galactose beziehungsweise LHP in einer Körnchengröße von 0,01 bis 0,1 mm untermischt ist.

8. Süßungsmittel nach einem der Ansprüche 1 bis 4, dadurch gekennzeichnet,

daß feinvermahlener kristalliner Saccharose feinvermahlenes galactosehaltiges Pulver zugesetzt ist,

daß dieses Galactose-Pulver gewonnen ist, indem auf

| 100 TGT | Galactose oder LHP |
|---|---|
| 150 bis 400 TGT | wasserlöslicher Füllstoff, vorzugsweise Glycin, und |
| 10 bis 40 TGT | Löslichkeitsbeschleuniger, vorzugsweise Natriumcarboxymethylcellulose |

in Wasser gut gemischt gelöst, eingetrocknet und dann gemahlen wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CA-A-1 225 864 (IMPERIAL BIOTECHNIQUES) * Ansprüche 1-34 * --- | 1,3,4 | A 23 L 1/236 A 23 C 9/12 C 13 F 3/00 C 13 K 13/00 |
| D,X | EP-A-0 184 121 (BIODYN) * Ansprüche 1-15; Seite 15, Zeilen 24-30 * ----- | 1,3,4 | |

RECHERCHIERTE
SACHGEBIETE (Int. Cl.4)

A 23 L
C 13 F
C 13 K
A 23 C

**Der vorliegende Recherchenbericht wurde für alle Patentansprüche erstellt**

| Recherchenort | Abschlußdatum der Recherche | Prüfer |
|---|---|---|
| DEN HAAG | 11-08-1989 | VAN MOER A.M.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument

EPO FORM 1503 03.82 (P0403)